# EUROPEAN PATENT APPLICATION

(11) **EP 3 135 768 A1**
(43) Date of publication of application: **01.03.2017**
(21) Application number: 15182601.3
(22) Date of filing: 26.08.2015
(51) Int. Cl.: C12Q 1/68

(54) **ZIC1 AND GHSR, MOLECULAR DIAGNOSTIC MARKERS FOR HPV-INDUCED INVASIVE CANCERS, NONHPV-INDUCED GYNAECOLOGICAL AND ANOGENITAL CANCERS AND THEIR HIGH-GRADE PRECURSOR LESIONS**

(71) Applicant: Self-screen B.V., 1098 RX Amsterdam (NL)
(72) Inventor: Hesselink, Albertus Theodorus, 1098 RX Amsterdam (NL)
(74) Representative: V.O.

(57) **Abstract**

The invention relates to method for detecting HPV-induced high-grade precancerous lesions, HPV-induced invasive cancers and nonHPV-induced gynaecological and anogenital cancers, said method comprising detection of hypermethylation in the ZIC1 and/or GHSR gene in a cell whereby such hypermethylation indicates the presence of HPV-induced precursor lesions with invasive potential, HPV-induced invasive cancers and nonHPV-induced gynaecological and anogenital cancers. The invention further comprises the use of the ZIC1 and/or GHSR gene in such a method and a testkit for the detection of ZIC1 and/or GHSR methylation.

## Description

### FIELD OF THE INVENTION

The invention relates to the field of cancer prevention and medical diagnostics; and is concerned with a molecular diagnostic marker for cancers, especially human papillomavirus (HPV)-induced invasive cancers and high-grade precursor lesions thereof, such as invasive cervical cancer and premalignant cervical lesions, nonHPV-induced gynaecological and anogenital cancers. In particular, the present invention relates to the use of the ZIC1 and GHSR genomic and regulatory sequence as marker for hrHPV-induced invasive cancers, nonHPV-induced gynaecological and anogenital cancers and their premalignant lesions with invasive potential.

### BACKGROUND OF THE INVENTION

Cancer of the uterine cervix is the fourth most common cancer in women world-wide and is responsible for approximately 250.000 cancer deaths a year.

Cervical squamous cell carcinoma development is characterized by a sequence of premalignant lesions, so-called cervical intraepithelial neoplasia (CIN), which are graded 1 to 3, referring to mild dysplasia (CIN 1), moderate dysplasia (CIN 2) and severe dysplasia/carcinoma *in situ* (CIN 3), respectively. CIN 1 is also referred to as low grade squamous intraepithelial lesion (LSIL) and CIN 2 and CIN 3 together as high grade squamous intraepithelial lesion (HSIL). For cervical adenocarcinoma, adenocarcinoma in situ (ACIS) is an established precursor lesion. In principle, these premalignant lesions are reversible, although the more severe the lesion, the lower the chance of spontaneous regression. Cervical cancer is considered a preventable disease because the premalignant stages can be detected by exfoliative cytology and treated relatively easily when necessary, with only minor side effects. Cervical screening is aimed to early diagnose the high-grade premalignant (i.e., CIN 2/3 and adenocarcinoma in situ) and treatable cancerous lesions, thereby reducing the mortality of invasive cervical cancer. General medical practice comprises the treatment of all women with morphologically confirmed CIN 2, CIN 3 and adenocarcinoma in situ, in order to prevent the development of cervical cancer.

Over the past decade it has been well established that cervical carcinogenesis is initiated by an infection with high-risk human papillomavirus (hrHPV). Expression of the viral oncogenes E6 and E7, which disturb the p53 and Rb tumor suppressor pathways, respectively, has been shown to be essential for both the onset of oncogenesis and the maintenance of a malignant phenotype. Therefore, testing for hrHPV appeared as an attractive, primary screening tool. However, consistent with a multistep process of carcinogenesis, additional alterations in the host cell genome are required for progression of an hrHPV infected cell to invasive cancer cell. Only a small proportion of women infected with high-risk HPV will develop high-grade premalignant cervical lesions (CIN 2/3) and, if left untreated, cervical cancer. In most women with premalignant cervical lesions the lesions regress spontaneously. Of the women who participate in population based screening, about 5-6% have a positive hrHPV test. However, only at maximum 20% of them (1% of the participating women) have ≥CIN 2/3. Therefore, primary screening by hrHPV testing will be accompanied with a substantial number of redundant follow-up procedures and unnecessary anxiety amongst women, unless markers can be applied to the cervical smears that allow stratification of hrHPV positive women for risk of ≥CIN 2/3 and ≥adenocarcinoma in situ.

A major challenge is to reduce the percentage of HPV test positive women to those that have clinically meaningful lesions. One mode is to use cytology as a secondary (so-called triage) test for hrHPV positive women. Still, this leaves a substantial number of hrHPV positive women with normal cytology (3.5% of the women in the screening population), of which still 10% have or acquire ≥CIN 3. Moreover, cytology is not an option for self-sampled cervico-vaginal specimens that can be taken at home, since these are not representative for the cytological status of the cervix. Another mode is to use HPV16/18 genotyping. This however leaves women with nonHPV16/18 types who are, although to a lesser extent, also at risk of ≥CIN 2/3 and ≥adenocarcinoma in situ. Therefore, there is a need for supplementary or alternative triage tools to stratify hrHPV positive women into those with and without ≥CIN 2/3 and ≥adenocarcinoma in situ.

Primary screening for cervical cancer using disease markers based on host cell changes in cancer genes provides a promising alternative provided that specificity and sensitivity is sufficiently high. This option is of particular interest for low and middle income countries, where quality-controlled cytology is absent and implementation of follow-up algorithms for HPV-positive women is complicated. In these countries self-sampling has shown to facilitate access to cervical screening (Laczano-Ponce et al., Lancet. 2011; 378: 1868-1873). In this sense it is extremely useful to have markers that also prevail in self-samples. It appears that there is a huge difference in the 'behaviour' of markers on vaginal smears that have been obtained by medical skilled personnel like doctors or nurses and on vaginal swabs that have been collected by the woman herself. It has appeared that many markers that would be suitable for doctor-provided samples are not useful in self-samples. The necessity to work with self-samples in stead of doctor samples is high in low and middle income countries since in those countries there is less medical personnel per capita and often there is a cultural problem by letting other persons taking a vaginal sample.

Endometrial cancer is the most common gynaecologic malignancy in many developed countries (Siegel et al., CA Cancer J. Clin., 64 (2014), pp. 9-29). Early stage endometrial cancer has a very good prognosis. Therefore, early detection will increase the chance of cure and avoid or reduce cumbersome and costly therapeutic intervention. As shedding of tumor cells in cervical scrapes has been demonstrated, early detection of endometrial cancer by non-invasive sampling is feasible. However, conventional cytology on cervical scrapes has a very low sensitivity for detection of endometrial cancer. Testing for molecular alterations, such as DNA methylation, associated with endometrial cancer, may provide a promising approach for early detection of endometrial cancer (de Strooper et al., J Clin Pathol. 2014;67(12):1067-71, Bakkum-Gamez et al. Gynecol Oncol. 2015;137(1):14-22). Similarly, testing for cancer specific DNA alterations in cervical scrapes for the detection of ovarian cancer has recently been proposed (Kindle et al., Sci Transl Med. 2013; 5(167):1-21).

### SUMMARY OF THE INVENTION

The inventors now have found a method for detecting HPV-induced invasive cancers, nonHPV-induced gynaecological and anogenital cancers, and their high-grade precancerous lesions wherein said method comprises the detection of hypermethylation in the ZIC1 and/or GHSR gene in a cell whereby such hypermethylation indicates the presence of HPV-induced precursor lesions with invasive potential, HPV-induced invasive cancers, nonHPV-induced gynaecological and anogenital cancers. Preferably, in such a method said HPV-induced high-grade precancerous lesion or HPV-induced invasive carcinoma is a high-grade premalignant cervical lesion or invasive cervical cancer, more preferably a high-risk HPV-induced invasive cancer. Preferably, said nonHPV-induced gynaecological cancer is a endometrial cancer. Preferably said nonHPV-induced anogenital cancer is a vulvar or penile cancer.

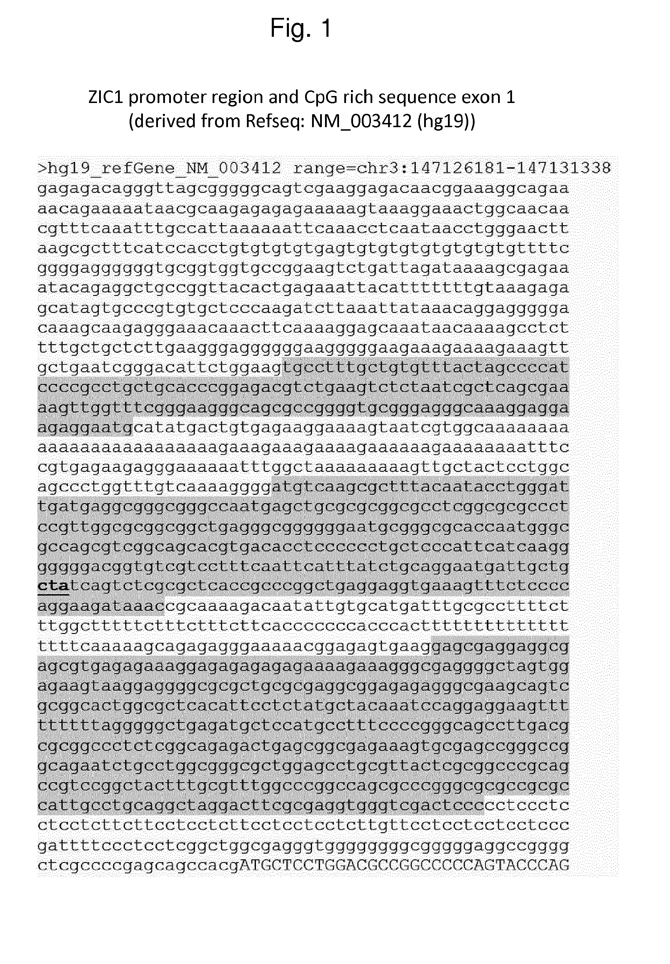

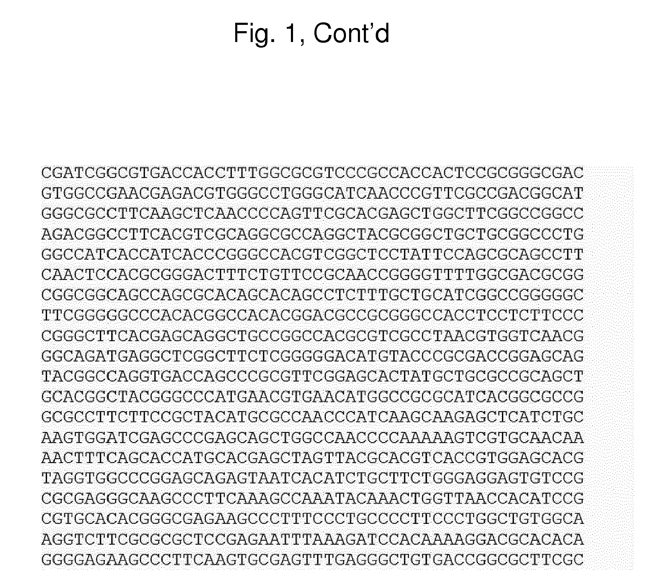

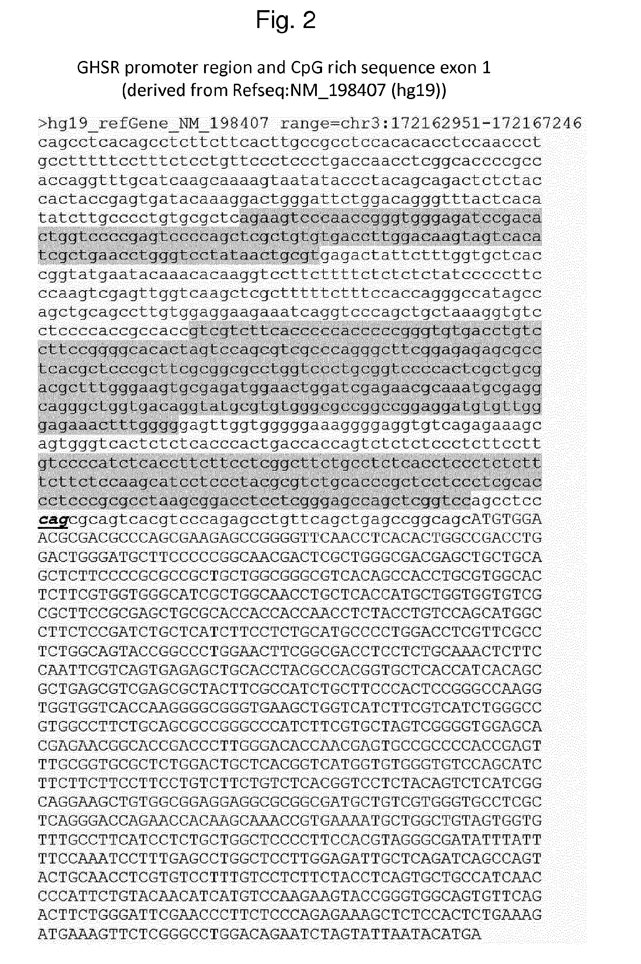

In a preferred embodiment of the invention the hypermethylation is detected in the CpG rich sequences as indicated in Figures 1 and 2.

The invention also relates to a method as defined above wherein said hypermethylation is an increased methylation of ZIC1 and/or GHSR CpG rich promoter and /or genomic sequences in the test cell as compared to the comparable normal cell.

In a preferred embodiment of the invention the detection of (hyper)methylation is performed by using a methylation sensitive restriction endonuclease, chosen from the group consisting of BssHII, MspI, NotI and HpaII. In another preferred embodiment of the invention the detection of (hyper)methylation is performed using nanotechnology. In an alternative preferred embodiment of the invention, the detection of (hyper)methylation is performed via a methylation specific PCR, which is based on bisulfite modification of DNA, followed by specific PCR reactions that target CpG rich sequences. Preferably in such a method the reagent is a nucleic acid probe or primer that binds to the nucleic acid as indicated in Figure 1 or 2, and more preferably said nucleic acid probe or primer has a detectable label.

In another embodiment of the invention the nucleic acid probe has a nucleotide sequence selected from the group consisting of:
a) a polynucleotide sequence capable of hybridizing under stringent conditions to the sequence ZIC1 as set forth in Figure 1 or to the sequence GHSR as set forth in Figure 2;
b) a polynucleotide having at least 70% identity to the polynucleotide of a);
c) a polynucleotide complementary to the polynucleotide of a); and
d) a polynucleotide comprising at least 15 bases of a nucleotide of a) or b).

Further preferred in the present method of the invention the methylation of both the ZIC1 and the GHSR gene is determined.

Also part of the invention is the use of ZIC1 and/or GHSR as a molecular diagnostic marker for the detection of HPV-induced invasive carcinoma, nonHPV-induced gynaecological or anogenital carcinoma, and their high-grade precancerous lesion,. Preferably, in such a use the methylation of said marker is predictive for the occurrence of said lesion or carcinoma.

The invention also comprises a kit of parts for use in a method of detecting HPV-induced invasive carcinoma, nonHPV-induced gynaecological or anogenital carcinoma and their high- grade precancerous lesion said kit comprising
- means for the detection of ZIC1 and/or GHSR methylation wherein said means comprise probes and/or primers specific for the ZIC1 nucleotide sequence of Figure 1 and/or the GHSR nucleotide sequence of Figure 2 and
- means for the detection of HPV infection, wherein said means comprise probes and primers specific for HPV.

### BRIEF DESCRIPTION OF THE DRAWINGS

Figure 1 shows the *ZIC1* 5' regulatory region and coding sequence. Transcription start is indicated in bold italic underlined, CpG islands shaded in grey and coding sequence is in upper case.
Figure 2 shows the *GHSR* 5' regulatory region and coding sequence. Transcription start is indicated in bold italic underlined, CpG islands shaded in grey and coding sequence is in upper case.

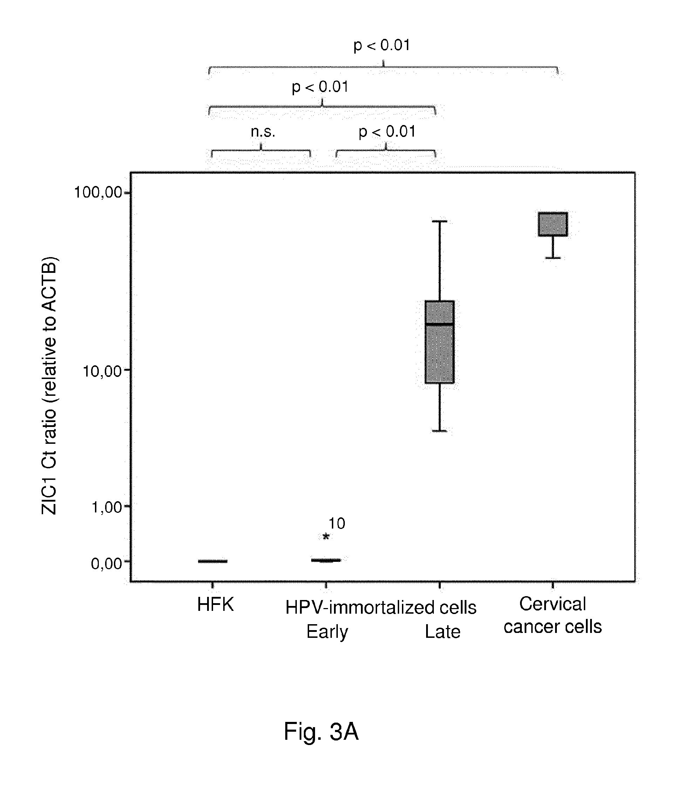

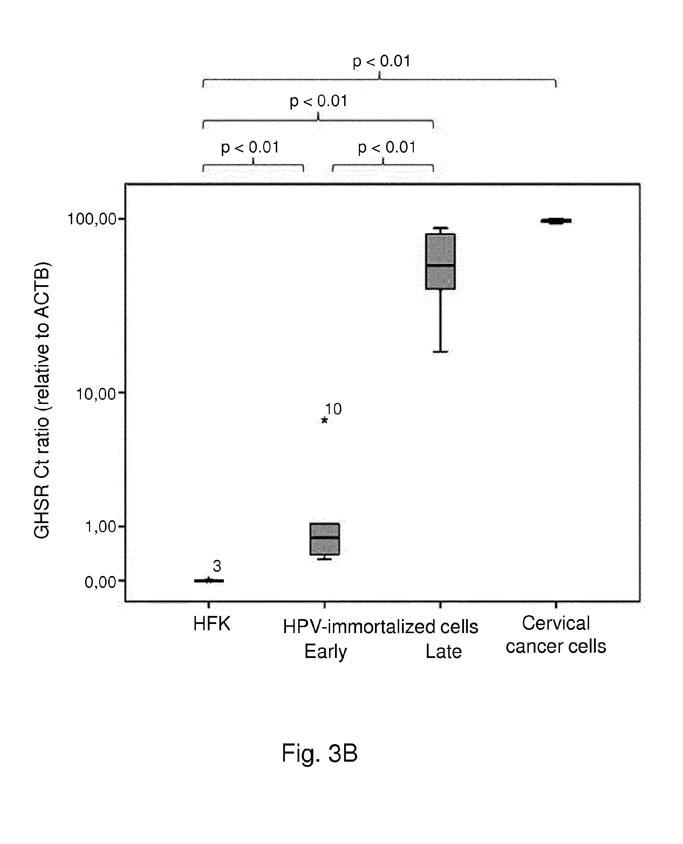

Figure 3 shows boxplots of the levels of ZIC1 and GHSR as measured by quantitative methylation specific PCR in primary keratinocytes, hrHPV-immortalized keratinocytes and cervical cancer cell lines. On the y-axes levels of methylated DNA are presented; on the x-axes the cell lines are grouped according to degree of transformation (primary keratinocytes, early passage HPV16 and HPV18 immortalized keratinocytes, late passage HPV16 and HPV18 immortalized keratinocytes and cervical cancer cells). The level of methylation of ZIC1 and GHSR increases with stage of transformation and is significantly increased in late passage HPV16 and HPV18 immortalized keratinocytes and cervical cancer cells compared to primary keratinocytes and early passage HPV16 and HPV18 immortalized keratinocytes. Significant differences in methylation levels between cell categories are indicated.

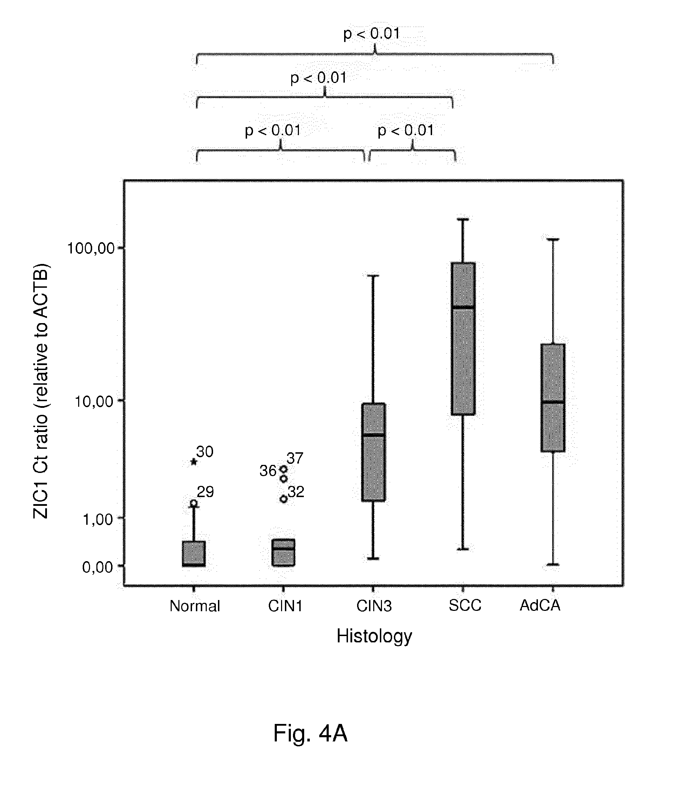

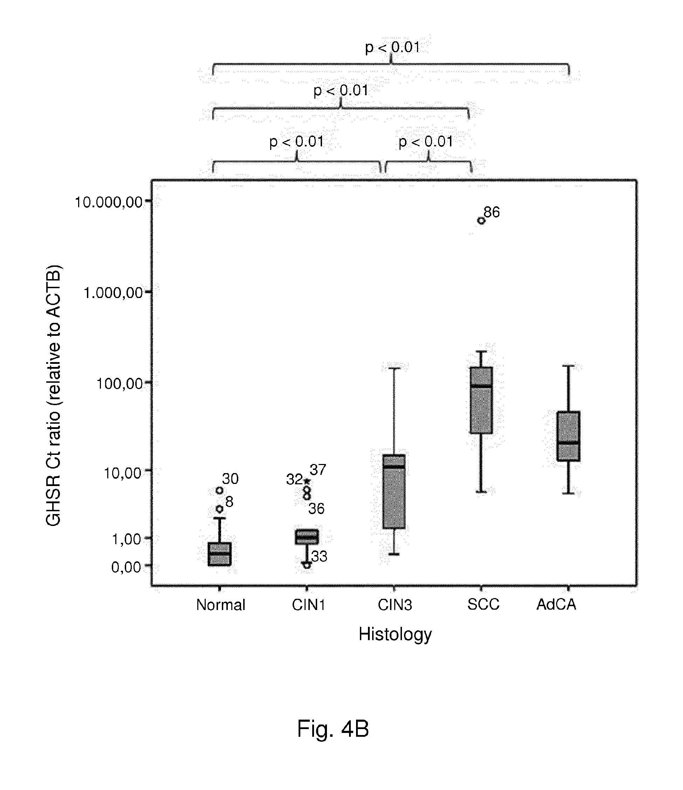

Figure 4 shows boxplots of the levels of ZIC1 and GHSR as measured by quantitative methylation specific PCR in cervical tissue specimens. On the y-axes levels of methylated DNA are presented; on the x-axes the samples are grouped for each disease stage and histotype. The level of methylation of ZIC1 and GHSR increases with stage of disease and is detected in (virtually) all SCC and AdCA. Significant differences in methylation levels between disease categories are indicated.

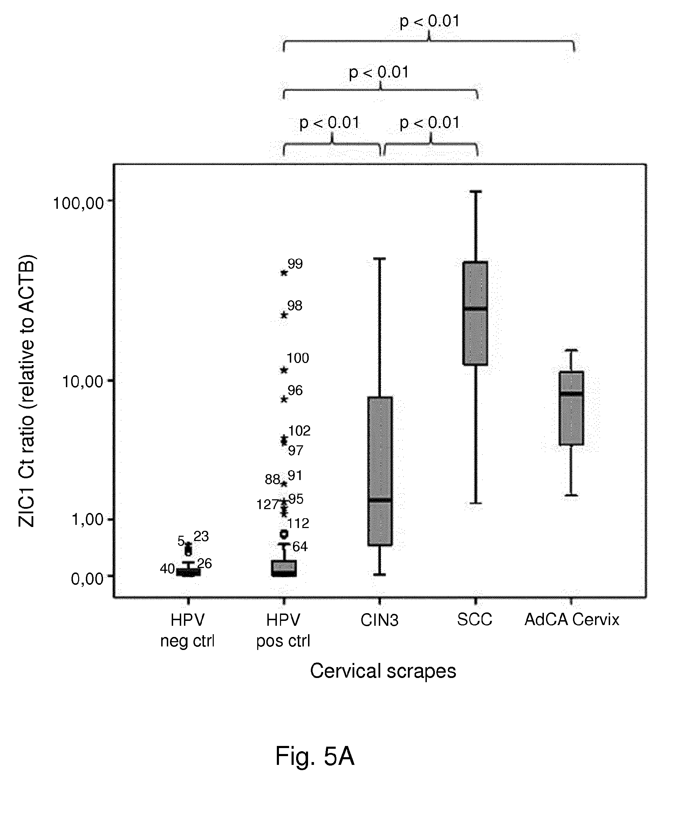

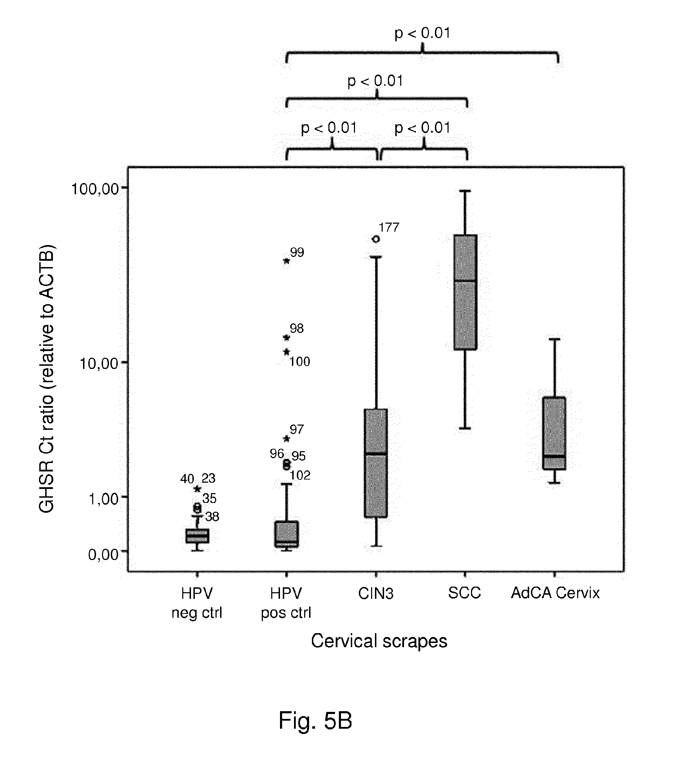

Figure 5 shows boxplots of the levels of ZIC1 and GHSR as measured by quantitative methylation specific PCR in cervical scrapes of women without cervical disease (including both HPV negative and HPV positive scrapes), women with CIN3, women with SCC and women with AdCA. On the y-axes levels of methylated DNA are presented; on the x-axes the samples are grouped for each disease stage and histotype. The level of methylation of ZIC1 and GHSR increases with stage of disease and is detected in all SCC and AdCA. Significant differences in methylation levels between disease categories are indicated.

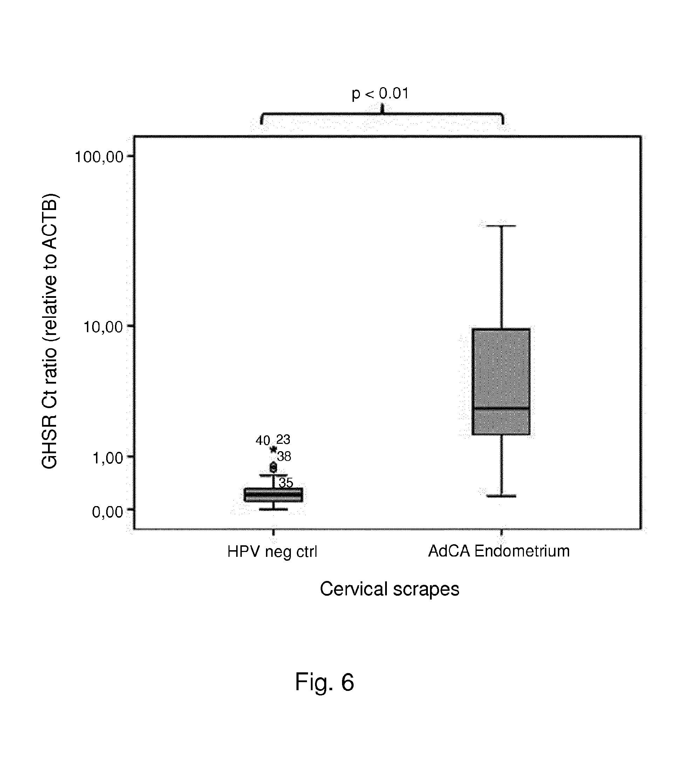

Figure 6 shows boxplots of the levels of GHSR as measured by quantitative methylation specific PCR in cervical scrapes of women with endometrial carcinomas. On the y-axes levels of methylated DNA are presented; on the x-axes the samples are grouped as scrapes of women without cervical disease and scrapes of women with endometrial cancer. GHSR methylation levels are significantly increased in virtually all women with endometrial carcinoma compared to controls.

### DETAILED DESCRIPTION OF THE INVENTION

The term "HPV-induced invasive cancer" refers to a carcinoma induced by high-risk HPV, which invades surrounding tissue. This includes all HPV-induced carcinoma histotypes, i.e., squamous cell carcinomas, adenocarcinomas, adenosquamous carcinomas and neuroendocrine carcinomas in relevant organs such as cervix, oral cavity, oropharynx, anus, rectum, penis, vulva, vagina, etc. It especially includes head and neck squamous cell carcinomas (HNSCC), cervical squamous cell carcinomas and cervical adenocarcinomas.

The term "invasive cervical cancer" refers to a cervical carcinoma invading surrounding tissue. This includes all carcinoma histotypes, i.e., squamous cell carcinomas, adenocarcinomas, adenosquamous cell carcinomas and neuroendocrine carcinomas.

The term "nonHPV-induced gynaecological or anogenital cancer" refers to endometrial cancer, ovarian cancer, vulvar cancer, vaginal cancer, anal cancer and penile cancer that are HPV-negative.

The terms "premalignant lesion" and "precursor lesion" refer to a stage in the multistep cellular evolution to cancer with a strongly increased chance to progress to a carcinoma. With classical morphology the pathologist is unable to predict in the individual patient which of these lesions will progress or regress. The current patent refers to a method, which can predict invasive cancer or a high-grade precursor lesion thereof.

The term "high-grade premalignant cervical lesion" refers to a stage in the multistep cellular evolution to cervical cancer with a strongly increased chance to progress to a cervical carcinoma. The term "capable of specifically hybridizing to" refers to a nucleic acid sequence capable of specific base-pairing with a complementary nucleic acid sequence and binding thereto to form a nucleic acid duplex.

A "complement" or "complementary sequence" is a sequence of nucleotides which forms a hydrogen-bonded duplex with another sequence of nucleotides according to Watson-Crick base-paring rules. For example, the complementary base sequence for 5'-AAGGCT-3' is 3'-TTCCGA-5'.

The term "stringent hybridization conditions" refers to hybridization conditions that affect the stability of hybrids, e.g., temperature, salt concentration, pH, formamide concentration and the like. These conditions are empirically optimised to maximize specific binding and minimize non-specific binding of the primer or the probe to its target nucleic acid sequence. The terms as used include reference to conditions under which a probe or primer will hybridise to its target sequence, to a detectably greater degree than other sequences (e.g. at least 2-fold over background). Stringent conditions are sequence dependent and will be different in different circumstances. Longer sequences hybridise specifically at higher temperatures. Generally, stringent conditions are selected to be about 5°C lower than the thermal melting point (Tₘ) for the specific sequence at a defined ionic strength and pH. The Tₘ is the temperature (under defined ionic strength and pH) at which 50% of a complementary target sequence hybridises to a perfectly matched probe or primer. Typically, stringent conditions will be those in which the salt concentration is less than about 1.0 M Na ion, typically about 0.01 to 1.0 M Na ion concentration (or other salts) at pH 7.0 to 8.3 and the temperature is at least about 30°C for short probes or primers (e.g. 10 to 50 nucleotides) and at least about 60°C for long probes or primers (e.g. greater than 50 nucleotides). Stringent conditions may also be achieved with the addition of destabilizing agents such as formamide. Exemplary low stringent conditions or "conditions of reduced stringency" include hybridization with a buffer solution of 30% formamide, 1 M NaCl, 1% SDS at 37°C and a wash in 2x SSC at 40°C. Exemplary high stringency conditions include hybridization in 50% formamide, 1 M NaCl, 1% SDS at 37°C, and a wash in 0.1x SSC at 60°C. Hybridization procedures are well known in the art and are described in e.g. Ausubel et al, Current Protocols in Molecular Biology, John Wiley & Sons Inc., 1994.

The term "oligonucleotide" refers to a short sequence of nucleotide monomers (usually 6 to 100 nucleotides) joined by phosphorous linkages (e.g., phosphodiester, alkyl and aryl-phosphate, phosphorothioate), or non-phosphorous linkages (e.g., peptide, sulfamate and others). An oligonucleotide may contain modified nucleotides having modified bases (e.g., 5-methyl cytosine) and modified sugar groups (e.g., 2'-O-methyl ribosyl, 2'-O-methoxyethyl ribosyl, 2'-fluoro ribosyl, 2'-amino ribosyl, and the like). Oligonucleotides may be naturally-occurring or synthetic molecules of double- and single-stranded DNA and double- and single-stranded RNA with circular, branched or linear shapes and optionally including domains capable of forming stable secondary structures (e.g., stem-and-loop and loop-stem-loop structures).

The term "primer" as used herein refers to an oligonucleotide which is capable of annealing to the amplification target allowing a DNA polymerase to attach thereby serving as a point of initiation of DNA synthesis when placed under conditions in which synthesis of primer extension product which is complementary to a nucleic acid strand is induced, i.e., in the presence of nucleotides and an agent for polymerization such as DNA polymerase and at a suitable temperature and pH. The (amplification) primer is preferably single stranded for maximum efficiency in amplification. Preferably, the primer is an oligodeoxy ribonucleotide. The primer must be sufficiently long to prime the synthesis of extension products in the presence of the agent for polymerization. The exact lengths of the primers will depend on many factors, including temperature and source of primer. A "pair of bi-directional primers" as used herein refers to one forward and one reverse primer as commonly used in the art of DNA amplification such as in polymerase chain reaction (PCR) amplification.

The term "probe" refers to a single-stranded oligonucleotide sequence that will recognize and form a hydrogen-bonded duplex with a complementary sequence in a target nucleic acid sequence analyte or its cDNA derivative.

DNA methylation is a biochemical process that is important for normal development in higher organisms. It involves the addition of a methyl group to the 5 position of the cytosine pyrimidine ring or the number 6 nitrogen of the adenine purine ring. DNA methylation at the 5 position of cytosine has the specific effect of reducing gene expression and has been found in every vertebrate examined. In adult somatic tissues, DNA methylation typically occurs in a CpG dinucleotide context.

Using a genome wide DNA methylation screen on cervical tissue specimens and HPV-immortalized cell lines it has now been found that the gene encoding Zic family member 1 (further referred to as *ZIC1*; Genbank Accession NM_003412) and the gene encoding Growth Hormone Secretagogue Receptor (further referred to as *GHSR;* Genbank Accession NM_198407) are targeted by DNA methylation in primary keratinocytes immortalized by full length HPV16 and HPV18 as well as in CIN3 lesions and cervical carcinomas, and that *ZIC1* and *GHSR* promoter methylation are important determinants of hr-HPV induced carcinogenesis. The *ZIC1* and *GHSR* genomic and regulatory sequences thus provide valuable markers to diagnose invasive cervical cancer and the high-grade precursor lesions thereof. Additionally, the present invention is suited to diagnose non-cervical hrHPV-associated invasive cancers and their high-grade precursor lesions. Moreover, GHSR methylation is additionally suited to diagnose endometrial and other nonHPV- induced gynaecological and anogenital cancers.

Cervical cancer is almost exclusively associated with human papillomavirus (HPV) infection. Human papillomaviruses, constitute a group of more than 150 types of viruses, as identified by variations in DNA sequence. The various HPVs cause a variety of cutaneous and mucosal diseases. HPVs are broadly classified into low-risk and high-risk types, based on their ability to induce malignant changes in infected cells. Low risk HPV types such as 1, 2, 4, 6, 11, 13 and 32 are primarily associated with benign lesions or common warts, while the high risk types, such as 16, 18, 31, 33, 35, 39, 45, 51, 52, 56, 58, 59, 66, and 68 are primarily associated with premalignant and malignant epithelial lesions. The high-risk HPV types have been found to cause invasive carcinoma of the uterine cervix, as well as invasive carcinoma elsewhere in the anogenital tract and/or head-neck region. Therefore, the present invention is not only suited to detect invasive cervical cancer and precursor stages thereof, but also other invasive cancers and corresponding precursor stages that are induced by HPV, particularly of the high-risk type. Thus, the present invention provides a method for the risk assessment of any HPV-induced high-grade premalignant lesion or invasive cancer.

Very suitable HPV-induced precursor lesions and invasive cancers in the context of the present invention are cervical precancerous lesions and invasive cervical cancers, but also precursor lesions and invasive cancers induced by high-risk HPV in other tissues such as oral cavity, oropharynx, anus, rectum, penis, vulva, vagina, etc.

As stated above, the GHSR marker also is capable of detecting nonHPV-induced precursor lesions and invasive cancers. Iin the context of the present invention such cancers preferably are endometrial cancer, ovarian cancer, vulvar cancer, vaginal cancer, anal cancer and penile cancer that are HPV-negative .

A test cell may be a (pre)neoplastic cell, a proliferating cervical cell, or any other cell wherein the presence of an HPV-induced precursor lesion with invasive potential, HPV-induced invasive cancer, nonHPV-induced gynaecological and anogenital cancer is to be detected.

The ZIC1 gene encodes a 48 kDa protein that functions as a transcription factor and is a member of the ZIC family of C2H2-type zinc finger proteins. Members of this family are important during development. ZIC1 is involved in neurogenesis. It plays important roles in the early stage of organogenesis of the CNS, as well as during dorsal spinal cord development and maturation of the cerebellum (reviewed by Grinberg and Millen, Clin Genet. 2005, 67(4):290-6). ZIC1 hypermethylation has been described in colorectal, gastric, ovarian and hepatocellular cancer (Gan et al., PLoS One. 2011, 6(2):e16916; Wang et al., Biochem Biophys Res Commun. 2009,379(4):959-63; Huang et al., Epigenetics. 2013, 8(6):624-34; Wang et al., Tumour Biol. 2014,35(8):7429-33). It has been mentioned as one of the factors that was found in a screen of hypermethylated genes in carcinoma *in situ* and cancer in a cervical swab (Wang et al., Cancer Med. 2015, 4(1):43-55) but only as one out of more than 2200 genes.

The *GHSR* gene, encodes a 41kDa protein that is a member of the G-protein coupled receptor family. The encoded protein may play a role in energy homeostasis and regulation of body weight (Howard et al., Science 1996,273(5277):974-7). GHSR hypermethylation has been described in cancers of the lung, breast, prostate, pancreas, colorectum, glioblastoma and B cell chronic lymphocytic leukemia (Moskalev et al., Oncotarget 2015,6(6):4418-27). Although it has been characterized as a pan-cancer marker, no link with HPV-induced cancers has yet been disclosed. It is surprising that this marker, that was found in other cancer types also appears to be a marker for HPV-induced cancer. It has been found in studies of head and neck squamous carcinoma (HNSCC), which are often caused by hrHPVs, that there is a difference in the methylome of HNSCCs that are HPV-induced and those that are not related to HPV (Sartor et al., 2011, Epigenetics 6(6):777-787). This was endorsed in other studies, where e.g. Colacino et al. (2013 PLOS One, 8(1):E54742 showed that hypermethylation of SPDEF, RASSF1, STAT5A, MGMT, ESR2, JAK3 and HSD17B12 is significantly associated with HPV-negative HNSCC. Similarly, Kostarelli et al. (J.Clin Investigation, 2013,123(6): 2488-2501) showed methylation of ALDH1A2, FKBP4, GDNF, OSR2, PROX1 and WIF1 to be significantly increased in HPV-negative HNSCC compared to normal mucosa, whereas these genes showed no hypermethylation in HPV-induced HNSCC. CDKN2A methylation as studied in oropharyngeal cancers was exclusively detected in HPV-negative cancers and absent in HPV-positive cancers (Taioli et al., 2009, BMC Cancer, 9: e354). Also in vulvar cancers methylations patterns differ between HPV-induced cancers and those not related to HPV, e.g. vulvar cancers associated with lichen sclerosus (LS) (Guerrero et al., 2010, Int J Cancer 28:2853-2864). For example, RASSF2A was found to be exclusively methylated in vulvar cancers associated with LS, that are HPV-negative. No RASSF2A methylation was detected in HPV-induced vulvar cancers.

The present inventors have now established that *ZIC1* and *GHSR* promoter methylation is a frequent event in cervical carcinomas of both squamous cell carcinoma, adeno-squamous carcinoma, adenocarcinoma and neuroendocrine carcinoma histotypes, and their high-grade precursor lesions. Most interestingly, the present inventors have shown that hypermethylation of the ZIC1 and GHSR promoter can be detected in cervical scrape samples and that this feature is able to predict the presence of a high-grade CIN lesion or invasive carcinoma. In addition, ZIC1 and GHSR promoter methylation could be detected in cervical-vaginal specimens collected by self-sampling and was found to be associated with the presence of an underlying high-grade CIN lesion or invasive cervical cancer.

Both hypermethylation of the ZIC1 and GHSR promoter can also be detected in HPV-positive and HPV-negative vulvar cancers and their high-risk precursor lesions.

Moreover, GHSR methylation is suited to diagnose endometrial and other nonHPV- induced gynaecological and anogenital cancers.

Accordingly, the present invention provides a method for detecting HPV-induced high-grade precancerous lesions and HPV-induced invasive cancers, nonHPV- induced gynaecological and anogenital cancers such as endometrial carcinoma, said method comprising detection of hypermethylation in the ZIC1 and/or GHSR gene in a cell whereby such hypermethylation indicates the presence of HPV-induced precursor lesions with invasive potential and HPV-induced invasive cancers and nonHPV-induced gynaecological and anogenital cancers such as endometrial carcinoma.

The test cell of the subject may comprise a cell from a sample of mucosal cells, such as cervical cells, and also other tissue such as oral cavity, oropharynx, penis, vulva, anus, rectum, endometrium, ovarium and other tissues wherein a precursor lesion or cancer associated with HPV or nonHPV- induced gynaecological, anogenital cancer or oropharyngeal cancer is to be detected. All such samples may be used as a sample in a method of the present invention. Preferably, a sample of a patient's cells comprise cervical cells or other epithelial cells of the anogenital or oropharyngeal tract as test cells. The cervical cells may e.g. be presented as a histological or cytological specimen. Cytological specimens comprise conventional cervical smears as well as thin layer preparations of cervical specimens and cervico-vaginal or vaginal specimens collected by self-sampling. Alternatively, cells may be presented in urine samples. A test cell wherein the present invention is especially advantageous over other known methods of detecting cancers in the cervix and adjacent tissues is a test cell obtained from a self-sample.

A method of the present invention is particularly suited for the detection of high-grade precancerous lesions and invasive cancers associated with ZIC1 and/or GHSR that are induced by high-risk HPVs or derived from the (female) anogenital tract. A method of detecting HPV-induced high-grade precancerous lesions with invasive potential, HPV-induced invasive cancers and nonHPV- induced gynaecological and anogenital cancer may comprise measuring the *ZIC1* and/or *GHSR* promoter.

Figure 1 shows the CpG-rich promoter region of the ZIC1 gene as well as the coding sequence.

Figure 2 shows the CpG-rich promoter regionof the GHSR gene as well as the coding sequence.

Detection of methylation is performed on nucleic acid, such as DNA.-The reagents that are used are typically a nucleic acid (DNA) probe or (PCR) primer or a restriction endonuclease, preferably a methylation sensitive restriction endonuclease for the detection of the presence of methyl groups on the test cell DNA.

The test cell component may be detected directly *in situ* or it may be isolated from other cell components by common methods known to those of skill in the art before contacting with the reagent (see for example, "Current Protocols in Molecular Biology", Ausubel et al. 1995. 4th edition, John Wiley and Sons; "A Laboratory Guide to RNA: Isolation, analysis, and synthesis", Krieg (ed.), 1996, Wiley-Liss; "Molecular Cloning: A laboratory manual", J. Sambrook, E.F. Fritsch. 1989. 3 Vols, 2nd edition, Cold Spring Harbor Laboratory Press)

Since examples presented show frequent methylation of the ZIC1 promoter, it is desirable to directly determine whether the *ZIC1* gene is hypermethylated. Similarly, it is also desirable to directly determine whether the *GHSR* gene is hypermethylated. In particular, the cytosine rich areas termed "CpG islands", which are primarily situated in the 5' regulatory regions of genes are normally unmethylated. The term "hypermethylation" includes any methylation of cytosine at a position that is normally unmethylated in the *ZIC1* or GHSR gene sequence (e. g. the *ZIC1* or *GHSR* promoter, first exon and first intronic sequence, see Figures 1 and 2, respectively). DNA methylation can be detected by the following assays currently used in scientific research:
- Methylation-Specific PCR (MSP), which is based on a chemical reaction of sodium bisulfite with DNA that converts unmethylated cytosines of CpG dinucleotides to uracil or UpG, followed by traditional PCR. However, methylated cytosines will not be converted in this process, and primers are designed to overlap the CpG site of interest, which allows one to determine methylation status as methylated or unmethylated.
- Whole genome bisulfite sequencing, also known as BS-Seq, which is a high-throughput genome-wide analysis of DNA methylation. It is based on aforementioned sodium bisulfite conversion of genomic DNA, which is then sequenced on a Next-generation sequencing platform. The sequences obtained are then re-aligned to the reference genome to determine methylation states of CpG dinucleotides based on mismatches resulting from the conversion of unmethylated cytosines into uracil.
- The HELP assay, which is based on restriction enzymes' differential ability to recognize and cleave methylated and unmethylated CpG DNA sites.
- ChIP-on-chip assays, which is based on the ability of commercially prepared antibodies to bind to DNA methylation-associated proteins like MeCP2.
- Restriction landmark genomic scanning, a complicated and now rarely-used assay based upon restriction enzymes' differential recognition of methylated and unmethylated CpG sites; the assay is similar in concept to the HELP assay.
- Methylated DNA immunoprecipitation (MeDIP), analogous to chromatin immunoprecipitation, immunoprecipitation is used to isolate methylated DNA fragments for input into DNA detection methods such as DNA microarrays (MeDIP-chip) or DNA sequencing (MeDIP-seq).
- Pyrosequencing of bisulfite treated DNA. This is sequencing of an amplicon made by a normal forward primer but a biatenylated reverse primer to PCR the gene of choice. The Pyrosequencer then analyses the sample by denaturing the DNA and adding one nucleotide at a time to the mix according to a sequence given by the user. If there is a mis-match, it is recorded and the percentage of DNA for which the mis-match is present is noted. This gives the user a percentage methylation per CpG island.
- Molecular break light assay for DNA adenine methyltransferase activity - an assay that relies on the specificity of the restriction enzyme DpnI for fully methylated (adenine methylation) GATC sites in an oligonucleotide labeled with a fluorophore and quencher. The adenine methyltransferase methylates the oligonucleotide making it a substrate for DpnI. Cutting of the oligonucleotide by DpnI gives rise to a fluorescence increase.
- Methyl Sensitive Southern Blotting is similar to the HELP assay, although uses Southern blotting techniques to probe gene-specific differences in methylation using restriction digests. This technique is used to evaluate local methylation near the binding site for the probe.
- Quantum-dot based methylation assay - an assay as described in Bailey, V. et al. (Genome Res. 19:1455-1461, 2009) in which the high specificity of MSP and the high sensitivity and simplicity of the quantum dot FRET (QD-FRET) technology (Zhang, C. et al., 2005, Nat. Mater. 4:826-831) is combined.
- DNA methylation detection using nanochip technology. This technique is able to detect DNA methylation at high sensitivity and specificity in minimal amounts of clinical material, without the need for bisulfite conversion and PCR amplification. Methods using solid states nanopores have been described by Shim, J. et al. (Sci. Rep. 3:1389, 2013). A device for lab on a chip technology is described in patent publication WO2009104967 (A1).

Hypermethylation preferably can be detected by restriction endonuclease treatment of the *ZIC1* or *GHSR* polynucleotide (gene) and Southern blot analysis. Any restriction endonuclease that includes CG as part of its recognition site and that is inhibited when the C is methylated, can be utilized. Methylation sensitive restriction endonucleases such as BssHII, MspI, NotI or HpaII, used alone or in combination, are examples of such endonucleases. Other methylation sensitive restriction endonucleases will be known to those of skill in the art.

An alternative preferred means to test for methylated sequences is a methylation specific PCR, which is also based on bisulfite modification of DNA, followed by specific PCR reactions that target CpG rich sequences.

A third preferred means to detect methylated sequences and to discriminate between methylated and unmethylated DNA is based on nanotechnology.

For purposes of the invention nucleic acid probe specific for ZIC1 or GHSR may be used to detect the presence of *ZIC1* or *GHSR* polynucleotide (using nucleic acid probe) in biological fluids or tissues. Oligonucleotide primers based on any coding sequence region and regulatory sequence region in the *ZIC1* or *GHSR* sequence are useful for amplifying DNA, for example by PCR.

When using PCR primers, nucleic acid probes or restriction endonucleases, the 5' regulatory region, first intronic sequence and coding sequence of the *ZIC1* or *GHSR* sequence (as specified in Figures 1 and 2 respectively) is analysed.

Any specimen containing a detectable amount of *ZIC1* or *GHSR* polynucleotide can be used. Preferred samples for testing according to methods of the invention include such specimens as (cervical or vaginal) scrapes, cervico-vaginal lavages or swabs, urine, blood and/or (cervical) biopsies and the like. Although the subject can be any mammal, preferably the subject is human.

Diagnostic methods for the detection of disorders, include methods wherein a sample for testing is provided, which sample comprises a cell preparation from cervical or other tissue. Preferably such samples are provided as smears or other cytological samples. Additional suitable samples include urine and blood.

A cell or tissue sample obtained from a mammal, preferably a human, is suitably pretreated to allow contact between the cellular DNA of a test cell comprised in said sample with a reagent that detects ZIC1 or GHSR and detects an alteration in the methylation of the ZIC1 or GHSR gene as compared to that of a comparable normal cell. Samples may be mounted on a suitable support to allow observation of individual cells. Examples of well-known support materials include glass, polystyrene, polypropylene, polyethylene, polycarbonate, polyurethane, optionally provided with layers to improve cell adhesion and immobilization of the sample, such as layers of poly-L-lysine or silane. Cervical smears or biopsies may for instance be prepared as for the Papanicolaou (Pap) test or any suitable modification thereof as known by the skilled person, and may be fixed by procedures that allow proper access of the reagent to the target component. In certain embodiments of the invention the cytological specimens are provided as conventional smear samples or thin layer preparations of cervical cells or liquid based cytology samples or any other kind of preparation known to those of skill in the art. If storage is required, routine procedures use buffered formalin for fixation followed by paraffin embedding, which provides for a well-preserved tissue infrastructure.

In one embodiment of a method of the invention an increased methylation of the *ZIC1* and/or *GHSR* promoter in the test cell is detected as compared to the comparable normal cell.

The present invention also provides a kit of parts as defined in the claims, for use in a method of detecting HPV-induced precursor lesions with invasive potential, HPV-induced invasive cancers and nonHPV- induced gynaecological and anogenital cancers associated with ZIC1 and/or GHSR in test cells of a subject. Such a kit may suitably comprise a brush or spatula to take a (cervical) scrape together with a container filled with collection medium to collect test cells. Alternatively, a sampling device consisting of an irrigation syringe, a disposable female urine catheter and a container with irrigation fluid will be included to collect cervical cells by cervico-vaginal lavage. Additionally, a container to collect urine is suitable, preferably to be used to collect first-void urine. A kit according to the present invention may comprise primers and probes for the detection of ZIC1 and/or GHSR promoter methylation.

A kit of parts according to the invention comprises means for the detection of *ZIC1* and/or *GHSR* promoter methylation, such as, methylation-sensitive restriction enzymes, or probes or primers capable of hybridising to the nucleotide sequence of Fig. 1 and/or Fig. 2.

In yet another alternative embodiment of a kit of the invention the means for the detection of *ZIC1* and/or *GHSR* promoter methylation may be combined with means for the detection of HPV infection, preferably for the detection of HPV infection of the high-risk type. Such means may comprise HPV-specific primers or probes, protein markers for HPV infection or even surrogate markers for HPV infection as are known in the art.

The present invention will now be illustrated by way of the following, non limiting examples.

### EXAMPLES

### Example 1. Discovery of ZIC1 and GHSR as methylation targets in HPV-induced transformation

A comprehensive analysis of genome-wide DNA methylation changes associated with HPV-induced transformation in vitro and cervical carcinogenesis in vivo has been conducted by means of MBD-sequencing of consecutive passages of HPV16 and HPV18-immortalized keratinocytes as well as cervical tissue specimens. Cell cultures included were 2 DNA isolates of primary human foreskin keratinocytes, 10 DNA isolates of keratinocytes transfected with full length HPV16 and HPV18 DNA (different passages of cell lines FK16A, FK16B, FK18A, FK18B; Steenbergen et al., Oncogene 1996, 13(6):1249-57), 2 DNA isolates of keratinocytes transduced with HPV16E6E7 (Steenbergen et al., J. Pathology 2013; 231(1):53-62) and the cervical cancer cell line SiHa. The cervical tissue specimens included consisted of 10 carcinomas, 12 high-grade cervical intraepithelial neoplasia(CIN2/3) and 3 low-grade cervical intraepithelial neoplasia(CIN1).
The MBD-sequencing method is based on the enrichment of CpG methylated fragments using methyl binding domains followed by massive parallel sequencing (Serre et al., Nucleic Acids Res. 2010;38(2):391-9). By mapping these fragments to a reference genome, the putatively methylated locus can be determined.

By this approach we identified ZIC1 and GHSR as novel methylation targets in HPV-induced transformation in vitro and cervical carcinogenesis in vivo.

Methylation of the ZIC1 and GHSR promoter regions was verified by quantitative Methylation Specific PCR (qMSP) in primary keratinocytes, early and late passages of HPV16 and HPV18-immortalized keratinocytes and cervical cancer cell lines. Primers and probes for qMSP are listed in Table 1. The housekeeping gene ß-actin (ACTB) was chosen as a reference for total DNA input measurement. Quantification was performed using the comparative Ct method (Schmittgen et al., Nat Protoc 2008, 3:1101-1108). The levels of methylation of ZIC1 and GHSR increase with stage of transformation and are significantly increased in late passage HPV16 and HPV18 immortalized keratinocytes and cervical cancer cells compared to primary keratinocytes and early passage HPV16 and HPV18 immortalized keratinocytes (Figure 3).

### Example 2: ZIC1 and GHSR promoter methylation are common events in high grade CIN lesions, cervical squamous cell carcinomas, adenosquamous carcinomas, adenocarcinomas and neuroendocrine carcinomas

Next, we analysed ZIC1 and GHSR promoter methylation in cervical tissue specimens by qMSP. Tissue samples tested included 30 normal cervical control samples, 13 CIN1 lesions, 29 CIN3 lesions and 24 cervical squamous cell carcinomas (SCC) . The housekeeping gene ß-actin (ACTB) was chosen as a reference for total DNA input measurement. Quantification was performed using the comparative Ct method (Schmittgen et al., Nat Protoc 2008, 3:1101-1108). We found that ZIC1 promoter methylation was significantly increased in CIN3 lesions and SCC compared to normal and CIN1 lesions. Moreover methylation levels were significantly increased in SCC compared to CIN3 lesions (Figure 4A). At a threshold setting resulting in 90% specificity, nearly all (96%) SCC and 79% of CIN3 lesions were positive for ZIC1 methylation. Next to cervical squamous cell carcinomas we also analysed ZIC1 promoter methylation in cervical adenocarcinomas. Adenocarcinomas, which constitute up to 20% of cervical carcinomas, are of particular interest as the incidence of cervical adenocarcinoma has remained the same or even increased in countries with a nation-wide cervical screening programme. This indicates that cervical adenocarcinoma and its glandular precursor lesion, i.e. adenocarcinoma in situ (ACIS), are frequently missed by cytology based screening. Based on comparative genetic and epigenetic studies between cervical squamous cell carcinomas and cervical adenocarcinomas it has been found that both tumor histotypes develop via distinct carcinogenenic pathways (Dong et al., 2001, Kang et al., 2005, Wilting et al., 2006, Henken et al., 2007). Consequently, most biomarkers enabling the detection of cervical squamous cell carcinoma do not necessarily detect cervical adenocarcinoma.

Interestingly, ZIC1 promoter methylation appeared to be an exception as, in contrast to most known markers, as ZIC1 methylation levels were increased in all but one (86%) AdCa tested (Figure 4A). Similar results have been obtained for cervical adenosquamous carcinomas and neuroendocrine carcinomas.

Therefore, ZIC1 promoter methylation appears to be a universal methylation marker for all cervical carcinoma histotypes.

Analysis of GHSR promoter methylation revealed significantly increased methylation levels increased in CIN3 lesions, SCC and AdCa compared to normal and CIN1 lesions. Moreover methylation levels were significantly increased in SCC compared to CIN3 lesions (Figure 4B). At a threshold setting resulting in 90% specificity, all (100%) SCC, all (100%) AdCa and 69% of CIN3 lesions were positive for GHSR methylation. Hence, GHSR promoter methylation also represents a universal methylation marker for all cervical carcinoma histotypes

### Example 3. Detection of ZIC1 and GHSR promoter methylation in hrHPV-positive cervical scrapes

From women participating in a population-based screening we studied cervical scrapes of hrHPV positive women in which CIN3 (n=56) was diagnosed, and hrHPV negative and positive women in whom at maximum CIN 1 was diagnosed (n=40 and n=87, respectively). Additionally, cervical scrapes of women diagnosed with SCC (n=23) and AdCa (n=3) of the cervix were tested. Cervical scrapes of these women were collected in preservation medium in which nucleic acids are well preserved.

ZIC1 methylation was significantly increased in scrapes of women with CIN3, SCC and AdCa compared to HPV negative and HPV positive controls. Methylation levels were also significantly increased in scrapes of women with SCC compared to women with CIN3 (Figure 5A). At a threshold setting resulting in 90% specificity, all (100%) scrapes of women SCC and AdCa were positive, and 56% of CIN3 lesions tested ZIC1 methylation positive. At 70% specificity, 86% of CIN3 lesions are detected.

GHSR methylation was significantly increased in scrapes of women with CIN3, SCC and AdCa compared to HPV negative and HPV positive controls. Methylation levels were also significantly increased in scrapes of women with SCC compared to women with CIN3 (Figure 5B). At a threshold setting resulting in 90% specificity, all (100%) scrapes of women SCC and AdCa were positive, and 58% of CIN3 lesions tested positive for GHSR methylation. At 70% specificity, 80% of CIN3 lesions are detected. Current data indicate that both ZIC1 and GHSR methylation analysis enable the detection of underlying cervical disease (CIN3+) in cervical scrapes at a high sensitivity and specificity. Particularly at high specificity settings both ZIC1 and GHSR outperform other published methylation markers and detect all cancers.

Thereby these genes provide promising triage markers in screening by primary HPV testing.

### Example 4: ZIC1 and GHSR promoter methylation as marker for primary screening

Methylation markers tested so far are not well suitable for use in primary screening due to a too low specificity at an acceptable sensitivity for CIN2/3 and cancer. For example markers or marker panels consisting of various combination of CADM1, MAL, miR-124 and/or FAM19A4 have a specificity of 70-75% at acceptable sensitivity of CIN2/3 (70%) and up to 100% detection of cervical cancer.

When evaluating the use of ZIC1 and GHSR methylation as marker for primary screening it was surprisingly found that both these markers have an extremely high sensitivity for detecting cancer at an extremely high specificity. At 100% specificity based on HPV-negative controls (and 90% specificity based on HPV-positive controls) ZIC1 methylation detects 100% of SCC, 100% of AdCa and 55% of CIN3 lesions.

At 95% specificity based on HPV-negative controls (and 90% specificity based on HPV-positive controls) GHSR methylation detects 100% of SCC, 100% of AdCa and 57% of CIN3 lesions. Hence primary screening by ZIC1 and/or GHSR enables the detection of virtually all cervical carcinomas at very high specificity (95-100%).

### Example 5: ZIC1 and GHSR promoter methylation in self-sampled specimens

We subsequently analysed self-sampled cervico-vaginal specimens collected using either a VibaBrush (Rovers Medical Devices, Oss, the Netherlands) or a Delphi-screener (Delphi BioScience BV, Scherpenzeel, The Netherlands). Self-collected samples are equivalent to physician-taken ones with respect to HPV testing (Brink et al. J Clin Microbiol 2006;44:2518-23). Testing for hrHPV in self-samples yields at least as much ≥CIN 2 lesions in this population as found by regular screening in a matched population of responder women (Bais et al., Int J Cancer: 2007, 120:1505-1510; Gök et al., BMJ 2010;340:c1040). However, also for HPV self-sampling there is a need for triage tools to stratify hrHPV positive women into those with and without ≥CIN 2/3 and ≥adenocarcinoma in situ. Whereas conventional cytology cannot be reliably performed on self-collected cervico-vaginal specimens (Brink et al. J Clin Microbiol 2006;44:2518-23), DNA methylation analysis can be applied to self-collected cervico-vaginal lavages (Eijsink et al., Gynecol Oncol 2011;120:280-3.). Importantly, the present findings show that the methylation markers GHSR and ZIC1 enable the detection of underlying CIN2+ not only when applied to self-collected cervico-vaginal lavage specimens but self-collected vaginal brush samples as well. The latter is a specimen type in which previous known markers often performed with low clinical sensitivity. Consequently, the ZIC1 and GHSR markers can be considered as pan-detection markers performing equal on physician-taken cervical smears, lavage-based self-samples and brush-based self-samples.

A series of 20 self-collected vaginal brush samples of hrHPV positive women without evidence of clinically meaningful disease in follow-up as well as 25 self-collected vaginal brush samples of hrHPV positive women with an abnormal follow-up smear and an underlying lesion ≥ CIN3 were tested by qMSP for ZIC1 and GHSR promoter methylation. Virtually all of self-collected vaginal brush samples of women that later were diagnosed with cancer tested positive of ZIC1 and GHSR methylation at a specificity of 70%. Additionally, more than half of self-collected vaginal brush samples of women that later were diagnosed with CIN3 tested positive for ZIC1 and GHSR promoter methylation.

Most interestingly, analysis of 24 self-collected cervico-vaginal lavage specimens of hrHPV positive women, revealed a detection of 100% of carcinomas at a specificity of up to 100% using ZIC1 or GHSR methylation analysis. ZIC1 methylation even detected 70% of CIN3 lesions at 90% specificity. Compared to known methylation markers applied to self-collected cervico-vaginal lavage specimens (lit.) the marker potential of ZIC1 in self-collected cervico-vaginal lavage specimens is remarkably high.

These data show that ZIC1 and GHSR promoter methylation analysis on self-sampled materials obtained from both self-collected vaginal brush samples and self-collected cervico-vaginal lavage specimens is well feasible and will improve the detection of underlying high-grade cervical disease. Most interestingly ZIC1 methylation analysis on self-collected cervico-vaginal lavage specimens is even suitable for primary screening

### (100% sensitivity for cervical cancer at 100% specificity)

### Example 6: GHSR promoter methylation in cervical scrapes of women with endometrial carcinoma

A total of 24 cervical scrapes of women with endometrial carcinoma were tested for GHSR methylation. Compared to cervical scrapes of hrHPV negative and positive women in whom at maximum CIN 1 was diagnosed (n=40 and n=87, respectively), GHSR methylation levels were significantly increased in women with endometrial carcinoma (Figure 6). At specific threshold settings at which ∼8% of controls tested positive, 80% of endometrial carcinomas were detected.

### Example 7: ZIC1 and GHSR promoter methylation in HPV-positive and HPV-negative vulvar cancers and their high-grade precursor lesions.

Like cervical precancerous lesions most vulvar precancerous lesions (vulvar intraepithelial neoplasia; VIN) result from an infection with HPV. Only a small subset of the lesions is at increased risk of progression to vulvar squamous cell carcinoma (VSCC). To determine whether ZIC1 and GHSR methylation can distinguish the lesion with a high risk of progression to cancer from those with a low cancer progression risk, the following tissue specimens were analysed by qMSP:

Normal vulva tissues (n=8; control vulvar tissues obtained from labiaplasty), HPV-positive VIN lesions of women that did not develop VSCC during at least 10 year follow-up (n=15; lesions with low risk of progression to cancer), HPV-positive VIN lesions of women with VSCC (n=15; lesions with high risk of progression to cancer) and vulvar squamous cell carcinomas (VSCC; n=50; including 38 HPV-negative and 12 HPV-positive cancers).

For both ZIC1 and GHSR methylation levels were significantly increased in HPV-positive VIN lesions of women with VSCC compared to HPV-positive VIN of women without VSCC. Moreover, methylation levels in VIN lesions associated with VSCC were similar to methylation levels detected in VSCC. This indicates that ZIC1 and GHSR methylation can be used for risk assessment of HPV-induced precancerous lesions of the vulva.

**Table 1 Primer and probe sequences (5'-3') used for ZIC1 and GHSR quantitative MSP analysis**

| | **Forward primer** | **Reverse primer** | **Probe** |
|---|---|---|---|
| ZIC1 | GGGCGGGTTAATGAGTTGC | TCACGTACTACCGACGCTAACG | CGCCGCGCCAACGAAAAAC |
| GHSR | GTTTGGTTTTTGCGGTTTTTATTC | CAACCCTACCTCGCATTTACG | CTCGATCCAATTCCATCTCGC ACTTCC |

## Claims

1. A method for detecting HPV-induced high-grade precancerous lesions and HPV-induced invasive cancers and nonHPV-induced gynaecological and anogenital cancers, said method comprising detection of hypermethylation in the GHSR and/or ZIC1 gene in a cell whereby such hypermethylation indicates the presence of HPV-induced precursor lesions with invasive potential, HPV-induced invasive cancers and nonHPV-induced gynaecological and anogenital cancers.

2. Method according to claim 1, wherein said HPV-induced high-grade precancerous lesion or HPV-induced invasive carcinoma is a high-grade premalignant cervical lesion or invasive cervical cancer.

3. Method according to claim 1 or 2, wherein said HPV-induced invasive cancer is a high-risk HPV-induced invasive cancer.

4. Method according to claim 1, wherein said nonHPV-induced gynaecological cancers is an endometrial cancer.

5. Method according to any one of the preceding claims, wherein said hypermethylation is detected in the CpG rich sequences as indicated in Figures 1 and 2.

6. A method of detecting HPV-induced high-grade precancerous lesion and HPV-induced invasive cancers and nonHPV-induced gynaecological or anogenital cancer according to any of claims 1-5, wherein said hypermethylation is an increased methylation of GHSR and/or ZIC1 CpG rich promoter and/or gene sequences in the test cell as compared to the comparable normal cell

7. Method according to any of claims 1-6, wherein the reagent is a methylation sensitive restriction endonuclease, chosen from the group consisting of BssHII, MspI, NotI and HpaII.

8. Method according to any of claims 1-6 wherein the method involves nanotechnology, preferably lab on a chip technology.

9. Method according to claim any of claims 1-6, wherein a methylation specific PCR is performed, which is based on bisulfite modification of DNA, followed by specific PCR reactions that target CpG rich sequences

10. Method according to claim 9, wherein the reagent is a nucleic acid probe or primer that binds to the nucleic acid as indicated in Figure 1 or 2.

11. Method according to claim 10, wherein said nucleic acid probe or primer has a detectable label.

12. Method according to claim 9, 10 or 11, wherein the nucleic acid probe has a nucleotide sequence selected from the group consisting of:
a) a polynucleotide sequence capable of hybridizing under stringent conditions to the sequence ZIC1 as set forth in Figure 1 or to the sequence GHSR as set forth in Figure 2;
b) a polynucleotide having at least 70% identity to one of the polynucleotides of a);
c) a polynucleotide complementary to one of the polynucleotides of a); and
d) a polynucleotide comprising at least 15 bases of one of the polynucleotides of a) or b).

13. Method according to any of claims 1 -12, wherein the methylation of both the GHSR and the ZIC1 gene is determined

14. Use of GHSR and/or ZIC1 as a molecular diagnostic marker for the detection of HPV-induced high-grade precancerous lesion or HPV-induced invasive carcinoma or nonHPV-induced gynaecological or anogenital cancer, preferably wherein the methylation of said marker is predictive for the occurrence of said lesion, carcinoma or cancer.

15. Kit of parts for use in a method of detecting HPV-induced high-grade precancerous lesion or HPV-induced invasive carcinoma or nonHPV-induced gynaecological or anogenital cancer, said kit comprising
- means for the detection of GHSR and/or ZIC1 methylation wherein said means comprise probes and/or primers specific for the ZIC1 nucleotide sequence of Figure 1 and/or the GHSR nucleotide sequence of Figure 2.
- means for the detection of HPV infection, wherein said means comprise probes and primers specific for HPV.
